(19) Europäisches Patentamt
European Patent Office
Office européen des brevets

(11) EP 1 044 980 A1

(12) **EUROPEAN PATENT APPLICATION**
published in accordance with Art. 158(3) EPC

(43) Date of publication:
**18.10.2000 Bulletin 2000/42**

(21) Application number: **98959142.5**

(22) Date of filing: **09.12.1998**

(51) Int. Cl.$^7$: **C07F 3/02**, C07C 41/06, C07C 43/225, C07C 63/04, C07C 65/21, C07C 51/15, C07C 323/62, C07C 319/20

(86) International application number:
**PCT/JP98/05571**

(87) International publication number:
**WO 99/29699 (17.06.1999 Gazette 1999/24)**

(84) Designated Contracting States:
**AT BE CH CY DE DK ES FI FR GB GR IE IT LI LU MC NL PT SE**

(30) Priority: **09.12.1997 JP 356197**
**12.12.1997 JP 36301997**

(71) Applicant:
**Ihara Chemical Industry Co., Ltd.**
**Taito-ku, Tokyo 110-0008 (JP)**

(72) Inventors:
- **YOSHIDA, Yasuo**
**Ihara-gun, Shizuoka 421-3306 (JP)**
- **HAMADA, Yusuke**
**Ihara-gun, Shizuoka 421-3306 (JP)**
- **MAGARIBUCHI, Kagetomo**
**Ihara-gun, Shizuoka 421-3306 (JP)**
- **TAKEUCHI, Hiroaki**
**Ihara-gun, Shizuoka 421-3306 (JP)**

(74) Representative: **HOFFMANN - EITLE**
**Patent- und Rechtsanwälte**
**Arabellastrasse 4**
**81925 München (DE)**

# (54) PROCESS FOR PRODUCING TOLUENE DERIVATIVES

(57) A toluene derivative of the general formula (2)

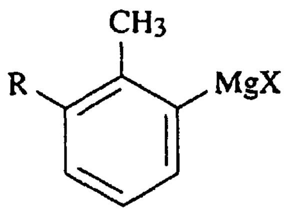

···(2)

wherein R denotes a fluorine atom, a chlorine atom, an alkoxy group, or an alkylthio group, and X denotes a halogen atom, is prepared by reacting metallic magnesium with a 2-chloro-6-substituted toluene derivative of the general formula (1)

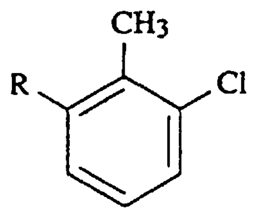

···(1)

wherein R is as defined above, in an ethereal solvent in the presence of a lower alkyl bromide.

EP 1 044 980 A1

## Description

## Technical Field

The present invention relates to a process for preparing a 2-alkoxy-6-chlorotoluene which is a toluene derivative serving as a useful intermediate for the production of physiologically active substances such as pharmaceuticals and agrochemicals, a 2-methyl-3-substituted phenylmagnesium halide which is a Grignard reagent corresponding to the 2-alkoxy-6-chlorotoluene, or an o-toluic acid which can be derived from the Grignard reagent.

## Background Art

A 2-alkoxy-6-chlorotoluene, or an o-toluic acid which is a compound derivable from this chlorotoluene derivative and having a substituent at the 3-position, is known to be useful as an intermediate for the production of physiologically active substances such as pharmaceuticals and agrochemicals.
In connection with the latter toluene derivative, an o-toluic acid having a substituent at the 3-position, e.g., 2-methyl-3-acetoxybenzoic acid, is used as an intermediate for anti-HIV (human immunodeficiency virus) drugs, as described in United States Patent 5484926 or WO 9521164. As Japanese Unexamined Patent Publication No. 6-199763 describes, the 2-methyl-3-methoxybenzoic acid is used as an intermediate for insecticides.
In connection with the 2-alkoxy-6-chlorotoluene, a toluene derivative, it is a known method to react sodium methylate with 2,6-dichlorotoluene in HMPA (hexamethylphosphoric triamide) as a solvent to form 2-methoxy-6-chlorotoluene, and lead this product to 2-chloro-6-hydroxytoluene without isolating it [Tetrahedron, Vol. 39, pp. 193-197 (1983)].
However, HMPA as the solvent used in this method is not favorable to use industrially because of its toxicity (potential carcinogenicity has also been pointed out). This method, if performed using a solvent other than HMPA, on the other hand, has keen insufficient in yield.
As a method for producing the latter toluene derivative, an o-toluic acid having a substituent at the 3-position, it has been known to react metallic magnesium with 2-chloro-6-methoxytoluene in a tetrahydrofuran solvent, with iodine as an initiator, to obtain 2-methyl-3-methoxyphenylmagnesium chloride (Grignard reagent), and further react carbon dioxide with the Grignard reagent to prepare 2-methyl-3-methoxybenzoic acid (Japanese Unexamined Patent Publication No. 58-170780).
However, a relevant portion (Referential Example 6) of the Japanese Unexamined Patent Publication No. 58-170780 fails to describe the amount of iodine added as the initiator to the reaction system. The inventors of the present application carried out the reaction for 24 hours under reflux, while varying the amount of iodine, under the conditions described in the above patent publication, as will be mentioned later on. When a trace amount of iodine (normally, a sufficient amount for a Grignard reagent formation reaction) was added, formation of a Grignard reagent was not confirmed. The inventors speculated that a reaction for formation of a Grignard reagent from an aromatic chlorine compound would proceed with difficulty, and that the presence of an alkyl group at the ortho-position relative to chlorine as in 2-chloro-6-methoxytoluene would make the progress of the reaction even more difficult based on its electronic and steric effects.
An object of the present invention is to provide a method for obtaining a Grignard reagent, with good reproducibility and in a high yield, from the corresponding 2-chloro-6-substituted toluene, an inert aromatic chlorine compound per se.
Another object of the invention is to provide a process for preparing an o-toluic acid having a substituent at the 3-position, with good reproducibility and in a high yield.
Still another object of the invention is to provide a process for preparing a 2-alkoxy-6-chlorotoluene, a useful starting material for the Grignard reaction, in a satisfactory yield without absolutely necessitating the use of a solvent which is toxic and poses difficulties in indusrial use.

Disclosure of the Invention

The inventors found that a 2-chloro-6-sustituted toluene derivative, an aromatic chlorine compound thus far regarded as being inert in a reaction with metallic magnesium, efficiently reacted with metallic magnesium in the copresence of a lower alkyl bromide in an ethereal solvent.
The process for preparing a toluene derivative according to the invention is based on this finding. More particularly, the process produces a toluene derivative of the following general formula (2)

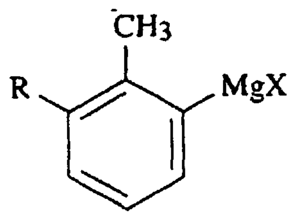

...(2)

wherein R denotes a fluorine atom, a chlorine atom, an alkoxy group, or an alkylthio group, and X denotes a halogen atom, by reacting metallic magnesium with a 2-chloro-6-substituted toluene derivative of the general formula (1)

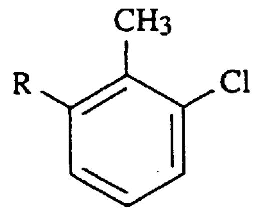

...(1)

wherein R is as defined above, in an ethereal solvent in the presence of a lower alkyl bromide.

The reason why the Grignard reaction is performed efficiently by the above-described process in the invention is estimated, according to the finding by the inventors, to be as follows: An alkylmagnesium bromide (alkyl Grignard reagent) formed from the alkyl bromide, and the 2-chloro-6-substituted toluene derivative cause a Grignard exchange reaction, which forms a 2-methyl-3-substituted phenylmagnesium halide and an alkyl halide. Further, the resulting alkyl halide and metallic magnesium reproduce an alkylmagnesium halide. Thus, a kihd of catalytic cycle is presumed to be established. (At this time, halogen ions may be interchanged.)

The inventors further proceeded with studies on the basis of the above findings. As a result, they found that the reaction of the Grignard reagent obtained in the above reaction (i.e., 2-methyl-3-substituted phenylmagnesium halide) with carbon dioxide made it possible to efficiently produce an o-toluic acid derivative having a substituent at the 3-position, and gave a process for preparation excellent in terms of the promotion of the reaction and the ease of industrial implementation.

The inventors further conducted extensive studies on a method for producing a 2-alkoxy-6-chlorotoluene which will be an embodiment of a starting material for the Grignard reaction. These studies led them to find that the 2-alkoxy-6-chlorotoluene was given easily and in a high yield by reacting an alcohol solution of a metal alcoholate (the alcoholate is not in a powdery state) with 2,6-dichlorotoluene in an aprotic polar solvent, while distilling off the alcohol.

The process for preparing a 2-alkoxy-6-chlorotoluene according to the invention is based on the above finding. In more detail, the process produces a toluene derivative of the general formula (3)

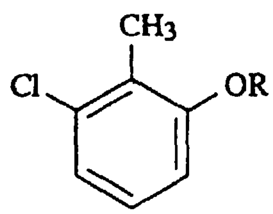

...(3)

wherein R denotes a lower alkyl group, by reacting 2,6-dichlorotoluene and an alcohol solution of a metal alcoholate in an aprotic polar solvent, while distilling off the alcohol, and then reacting an alkylating agent with the resulting product.

As described above, the production of a 2-alkoxy-6-chlorotoluene according to the invention is characterized by reacting 2,6-dichlorotoluene with the use of an alcohol solution of a metal alcoholate (not a metal alcoholate in a powdery state) in an aprotic polar solvent, while distilling off the alcohol. By so doing, it becomes easy to perform the reaction with high efficiency, while resolving the drawback of a powdery metal alcoholate which has so far been used (this type of alcoholate is usually a very hygroscopic powder difficult to use on a large scale, such as in industrial use).

## Best Mode for Carrying Out the Invention

The present invention will now be described in detail. In the following description, "parts" and "%" which represent quantitative proportions are on a weight basis unless otherwise specified.

(Grignard Reagent)

In the invention, a 2-methyl-3-substituted phenylmagnesium halide (Grignard reagent) of the general formula (2)

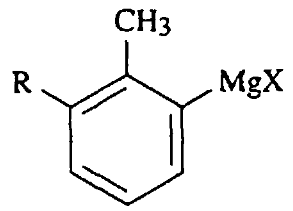

...(2)

wherein R denotes a fluorine atom, a chlorine atom, an alkoxy group, or an alkylthio group, and X denotes a halogen atom, is obtained by reacting metallic magnesium with a 2-chloro-6-substituted toluene derivative (i.e., o-chlorotoluene having a substituent at the 6-position) of the general formula (1)

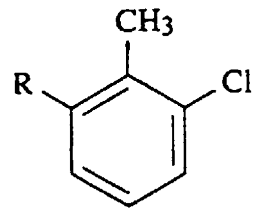

...(1)

wherein R denotes a fluorine atom, a chlorine atom, an alkoxy group, or an alkylthio group, in an ethereal solvent in the presence of a lower alkyl bromide (Grignard reaction).

(2-Alkoxy-6-Chlorotoluene)

In the invention, a 2-alkoxy-6-chlorotoluene of the general formula (3)

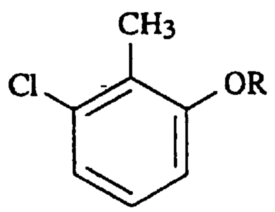

...(3)

wherein R denotes a lower alkyl group, which is an embodiment of the 2-chloro-6-substituted toluene derivative (1), the starting material for the Grignard reaction, can be obtained, for example, by reacting an alcohol solution of a metal alcoholate (the alcoholate is not in a powdery state) with 2,6-dichlorotoluene in an aprotic polar solvent, while distilling off the alcohol, and then alkylating the resulting product with an alkylating agent.

(Aprotic Polar Solvent)

In the production of the 2-alkoxy-6-chlorotoluene (3) in the invention, a publicly known aprotic polar solvent can be used without restriction. The use of HMPA (defective because of toxicity) as in the prior art is not indispensable

in the invention.

Examples of the aprotic polar solvent usable in the process of the invention include sulfur-containing aprotic polar solvents such as sulfolane ($TMSO_2$) and dimethyl sulfoxide (DMSO); and amidic aprotic polar solvents such as N,N-dimethylformamide (DMF), N,N-dimethylacetamide (DMAC), 1,3-diemthyl-2-imidazolidinone (DMI), N-methylpyrrolidone (NMP), and tetramethylurea.

In terms of reactivity, dimethyl sulfoxide and 1,3-diemthyl-2-imidazolidinone are preferred. In terms of low toxicity, and ease of separation of the product from the solvent, dimethyl sulfoxide is particularly preferred.

When dimethyl sulfoxide is used as the aprotic polar solvent in the invention, the reaction proceeds smoothly. As other advantages, dimethyl sulfoxide and the desired product (2-alkoxy-6-chlorotoluene) can be separated from each other with high efficiency simply by adding an aliphatic or alicyclic hydrocarbon solvent, without using water, after completion of a 2-alkoxy-6-chlorotoluene synthesis reaction, to perform extraction, followed by phase separation. Furthermore, it becomes easy to recover and recycle dimethyl sulfoxide as the solvent.

The amount of the aprotic polar solvent used is not restricted. However, in terms of the balance between the ease of an operation in the reaction, such as stirring, and the efficiency of the reaction, it is preferred to use the aprotic polar solvent in a range of 0.1 to 2.0 liters (further, 0.3 to 1.0 liter) per mol of 2,6-dichlorotoluerre, the starting material.

(Metal Alcoholate)

In the production of the 2-alkoxy-6-chlorotoluene in the invention, an alcohol solution of a metal alcoholate is used.

The metal constituting the metal alcoholate is not restricted, as long as it is capable of forming a metal alcoholate-in an alcohol. However, it is preferred to use an alkali metal, such as sodium or potassium, because of high reactivity, availability and handling, and a low price.

The alcohol constituting the metal alcoholate is not restricted, as long as it is capable of forming a metal alcoholate in the alcohol. However, it is preferred to use a lower alcohol, because of reactivity, easy separation of the product from the alcohol, and easy acquisition as a metal alcoholate. Here, the term "lower" about alcohol means that the number of carbon atoms is from 1 to 6. That is, the structure of the alkyl group contained in the metal alcoholate or the lower alcohol may have a straight chain and/or a branched chain (or a combination thereof).

In the invention, in terms of simplification of the process and reactivity with the 2-alkoxy-6-chlorotoluene, a characteristic of the invention, it is preferred to convert the metal, the raw material for the metal alcoholate, into a metal alcoholate in the alcohol, and then use the resulting metal alcoholate as "an alcohol solution of the-metal alcoholate" for production of the 2-alkoxy-6-chlorotoluene, i.e., use the metal alcoholate in the unchanged state containing the solvent alcohol without being dried.

Examples of the metal alcoholate usable in the process of the invention include straight chain or branched chain lower alcoholates, such as sodium methylate (methanol solution), sodium ethylate (ethanol solution), sodium isopropoxide (isopropanol solution), sodium n-propoxide (n-propanol solution), sodium n-butoxide (n-butanol solution), sodium isobutoxide (isobutanol solution), potassium methylate (methanol solution), potassium ethylate (ethanol solution), potassium isopropoxide (isopropanol solution), potassium n-butoxide (n-butanol solution), and potassium isobutoxide (isobutanol solution).

In the invention, the metal alcoholate is preferably used in a proportion of 1.0 to 4.0 mols (further 1.2 to 3.0 mols) per mol of 2,6-dichlorotoluene, the starting material.

If a commercially available metal alcoholate dry powder (e.g. a sodium ethoxide powder), for example, is used, it is usually difficult to dissolve this powder in an aprotic polar solvent. Thus, the use of the powder inevitably lowers the reaction rate (see "Comparative Examples" to be offered later on).

(Reaction Procedure)

A preferred embodiment of the reaction procedure for the production of the 2-alkoxy-6-chlorotoluene in the invention will be described below.

In this embodiment, 2,6-dichlorotoluene, themetal alcoholate (alcohol solution), and the aprotic polar solvent are mixed at atmospheric pressure. With mere stirring, the mixture is heated, and while separating alcohol by distilling it off or by other means, the reaction is caused to proceed. After the distilling off of alcohol is completed (after the temperature of the reaction mixture rises to a predetermined temperature), heating with stirring may be continued, where necessary, with the predetermined temperature being maintained. To perform this reaction, pressurization is normally unnecessary. If desired, the reaction may be performed, with at least one of the 2,6-dichlorotoluene and the alcohol solution of the metal alcoholate being added dropwise. In this invention, it becomes easy to increase the yield of the desired product, by setting a temperature at which the starting material and the metal alcoholate are reacted, with alcohol being distilled off.

In distilling off alcohol in the above procedure, the use of a rectifying means is preferred to perform the distilling off step stably. As such a rectifying means, a publicly known means (e.g., a rectification column packed with Raschig rings) can be used without restriction.

The reaction temperature for the subject reaction may usually be set in a range from a temperature higher than the boiling point of the alcohol dissolving the metal alcoholate used, to a temperature lower than the boiling point of the aprotic polar solvent used or the boiling point of the product. Usually, it is preferred for the reaction temperature to be 110 to 170°C (further, 130 to 160°C).

The reaction time is not restricted. From the aspect of the balance between the yield and the reaction efficiency, it is usually preferred for the reaction time to be 2 to 24 hours (further, 3 to 12 hours).

At a time when the starting material (2,6-dichlorotoluene) has nearly disappeared (the disappearance is confirmed by gas chromatography, high performance liquid chromatography or TLC (thin-layer chromatography)), 2-chloro-6-hydroxytoluene often occurs as a by-product in the form of a metallic salt in addition to the desired 2-alkoxy-6-chlorotoluene. This by-product, 2-chloro-6-hydroxytoluene, may have resulted, because the desired product (2-alkoxy compound) further reacted with the metal alcoholate and was thereby dealkylated. This 2-chloro-6-hydroxytoluene can be converted almost entirely into the desired 2-alkoxy-6-chlorotoluene by adding an alkylating agent in the same reactor to the resulting reaction mixture upon completion of the reaction with the metal alcoholate to alkylate the 2-chloro-6-hydroxytoluene.

As will be described later on, when the aprotic polar solvent is dimethyl sulfoxide, it is easy to extract the desired product with an aliphatic or alicyclic hydrocarbon solvent, and separate the product, without adding water to the reaction mixture after completion of the reaction. Thus, the use of dimethyl sulfoxide as the solvent is preferred.

(Alkylating Agent)

The alkylating agent used in the invention is not restricted, and can be suitably selected from publicly known alkylating agents according to the type of the metal alcoholate used.

Examples of the alkylating agent are alkyl halides, such as methyl iodide, methyl bromide, methyl chloride, ethyl iodide, ethyl bromide, ethyl chloride, n-propyl iodide, n-propyl bromide, n-propyl chloride, isopropyl iodide, isopropyl bromide, isopropyl chloride, n-butyl iodide, n-butyl bromide, n-butyl chloride, isobutyl iodide, isobutyl bromide, isobutyl chloride, sec-butyl iodide, sec-butyl bromide, and sec-butyl chloride; dialkyl sulfates such as dimethyl sulfate and diethyl sulfate; and alkyl p-toluenesulfonates such as methyl p-toluenesulfonate and ethyl p-toluenesulfonate. If desired, two or more 2-chloro-6-alkoxytoluenes can be prepared in the same system, by selecting the alkylating agent suitably.

In terms of the influence on the Earth's atmosphere (the ozone layer), the use of the alkylating agent other than methyl bromide is preferred.

(Alkylation)

Normally, the alkylation with the alkylating agent described above can be sufficiently performed simply by adding the alkylating agent to the reaction mixture after the reaction between the 2,6-dichlorotoluene and the metal alcoholate is completed. Where necessary, cooling, or heating with stirring may be performed during the alkylation.

The 2-chloro-6-hydroxytoluene, formed as a by-product in the form of a metallic salt in the previous reaction, can be converted almost entirely into a 2-chloro-6-alkoxytoluene by the alkylation.

The amount of the alkylating agent used can be determined based on the amount of 2-chloro-6-hydroxytoluene (can be grasped, for example, from the total area ratio by gas chromatography) which is formed by sampling the reaction mixture at the time of completion of the reaction between 2,6-dichlorotoluene and the metal alcoholate, and treating the sample with an acid (hydrochloric acid, sulfuric acid, or the like). The amount of the alkylating agent used is preferably from an equimolar amount to a slightly excess amount relative to the amount of 2-chloro-6-hydroxytoluene determined by gas chromatography.

Normally, it is preferred to use the alkylating agent in a range of from 0.3 to 0.7 mol (further, 0.4 to 0.6 mol) per mol of 2,6-chlorotoluene, the starting material.

The reaction temperature for the alkylation may generally be room temperature (25°C) or higher, but lower than the boiling point of the solvent or the product. Normally, the range of 20 to 120°C (further, 25 to 100°C) is preferred. The reaction time is preferably 0.5 to 5 hours (further, 1 to 3 hours).

(Copresence of Base)

In performing the alkylation, the copresence of a base in the reaction system may be able to achieve an increase in the yield of the desired product.

The base used for this purpose is, for example, the same metal alcoholate as used in the previous reaction.

Alternatively, an alkali metal hydroxide, such as potassium hydroxide or sodium hydroxide, may be used.

It is sufficient that the amount of the base used be usually about 0.05 to 0.2 equivalent based on 2,6-dichlorotoluene.

(Separation and Purification)

In the invention, after the alkylation is completed, the resulting 2-alkoxy-6-chlorotoluene may be separated and purified, if desired.

For this separation/purification step, an exemplary method is to add water to the resulting reaction mixture, and extract the product with an organic solvent. As a result, the aprotic polar solvent used in the reaction can be driven into the aqueous phase, and separation from the desired product can be facilitated. Direct rectification of the reaction mixture can also result in the separation and purification of the desired product.

Examples of the organic solvent that can be preferably used in the separation and purification are ethers such as diethyl ether; halogenatcd hydrocarbons such as dichloromethane; aromatic hydrocarbons such as benzene and toluene; and aliphatic or alicyclic hydrocarbons including n-pentane, n-hexane, n-heptane, n-octane, petroleum ether, cyclopentane, cyclohexane, and cycloheptane.

In the invention, depending on a combination of the aprotic polar solvent and the extraction solvent, it is possible to transfer the desired product from an aprotic polar solvent layer to an extraction solvent layer, without adding water. That is, when there is used an extraction solvent which has low mutual solubility relative to the aprotic polar solvent and which has sufficient affinity for the desired 2-alkoxy-6-chlorotoluene, the desired product can be obtained merely by removing inorganic salts from the reaction mixture by filtration or the like, adding the extraction solvent to the filtrate, stirring the mixture simply, separating the resulting phases to obtain the extraction solvent phase, washing the extraction solvent phase with water, and then recovering the extraction solvent.

Furthermore, by distilling the aprotic polar solvent phase that has remained upon extraction, the aprotic polar solvent can be recovered (recycled) conveniently and at a high recovery ratio. The aprotic polar solvent usable in performing the process of the invention with the intention to separate and purify the desired product must fulfill the following requirements: stability of the solvent, ease of progress of the reaction, low solubility in the extraction solvent, and minimum of toxicity which will jeopardize its industrial use. In the light of these requirements, the use of dimethyl sulfoxide is the most suitable. The extraction solvent can be used alone or as a mixture, where necessary.

As the extraction solvents used in combination, aliphatic or alicyclic hydrocarbons including, for example, n-pentane, n-hexane, n-heptane, n-octane, petroleum ether, cyclopentane, cyclohexane, methylcyclohexane, ethylcyclohexane, and cycloheptane can be used preferably.

In performing the process of the invention on a industrial scale, it is preferred to carry out as far as the alkylation with the use of dimethyl sulfoxide, then remove inorganic salts, and directly extract the desired product from the reaction mixture with the use of an aliphatic or alicyclic hydrocarbon. According to this method, even when 2-chloro-6-hydroxytoluene remains unremoved, the incorporation of the 2-chloro-6-hydroxytoluene into the extraction solvent comprising a hydrocarbon can be suppressed, and the procedure becomes easy and convenient.

(Grignard Reaction)

As described above, in the invention, a 2-methyl-3-substituted phenylmagnesium halide (Grignard reagent) of the general formula (2)

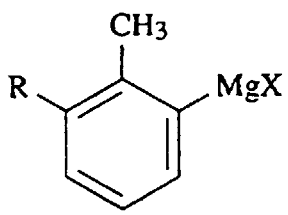

...(2)

wherein R denotes a fluorine atom, a chlorine atom, an alkoxy group, or an alkylthio group, can be obtained by reacting metallic magnesium with a 2-chloro-6-substituted toluene derivative (i.e., o-chlorotoluene having a substituent at the 6-position) of the general formula (1)

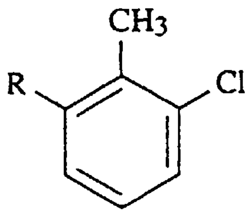

···(1)

wherein R denotes a fluorine atom, a chlorine atom, an alkoxy group, or an alkylthio group, in an ethereal solvent in the presence of a lower alkyl bromide.

(2-Chloro-6-Substituted Toluene Derivative)

As the 2-chloro-6-substituted toluene derivative of the general formula (1), which should be used as the starting material in the invention, there can be used an o-chlorotoluene derivative in which the substituent expressed as R is a fluorine atom; a chlorine atom; a straight chain or branched chain alkoxy group having 1 to 6 carbon atoms, concretely, a methoxy group, arr ethoxy group, an n-propoxy group, an isopropoxy group, an n-butoxy group, an isobutoxy group, a t-butoxy group, an n-pentyloxy group, or an n-hexyloxy group; or a straight chain or branched chain alkylthio group having 1 to 6 carbon atoms, concretely, a methylthio group, an ethylthio group, an n-propylthio group, an isopropylthio group, an n-butylthio group, an isobutylthio group, a t-butylthio group, an n-pentylthio group, or an n-hexylthio group.

Examples of the 2-chloro-6-substituted toluene derivative of the general formula (1) having the substituent R are dihalogenotoluenes such as 2,6-dichlorotoluene and 2-chloro-6-fluorotoluene; 2-chloro-6-alkoxytoluenes such as 2-chloro-6-methoxytoluene, 2-chloro-6-ethoxytoluene, 2-chloro-6-propoxytoluene, 2-chloro-6-isopropoxytoluene, and 2-chloro-6-butoxytoluene; and 2-chloro-6-alkylthiotoluenes such as 2-chloro-6-methylthiotoluene, 2-chloro-6-ethylthiotoluene, and 2-chloro-6-propylthiotoluene.

(Lower Alkyl Bromide)

In the foregoing Grignard reaction, a lower alkyl bromide having 1 to 4 carbon atoms can be used. The lower alkyl group constituting the lower alkyl bromide is not restricted, and may be of a straight chain or branched chain (or a combination of these) structure. More concretely, methyl bromide, ethyl bromide, n-propyl bromide, i-propyl bromide or n-butyl bromide, for example, is usable. Of them, i-propyl bromide is preferably used, because of the ease of handling (liquid at room temperature), and the stability of the resulting Grignard reagent. (In this case, however, i-PrCOOH which forms as a by-product upon reaction with carbon dioxide is smelly). As stated earlier, the use of a lower alkyl bromide other than methyl bromide is preferred from the point of view of the Earth's environment.

(Metallic Magnesium)

Metallic magnesium to be used in the invention is not restricted. Metallic magnesium as used in an ordinary Grignard reaction (e.g., ribbony or powdery metallic magnesium) is usable.

The amount of the metallic magnesium is preferably in excess of the amount of the 2-chloro-6-substituted toluene derivative of the general formula (1) by an amount in which a lower alkylmagnesium bromide is formed, in order to keep the reproducibility of the reaction fully and obtain an appropriate production cost. Although depebdent on the amount of the lower alkyl bromide used in the invention, the amount of the metallic magnesium used is preferably in the range of 1.05 to 2 mols (further, 1.1 to 1.5 mols) per mol of the 2-chloro-6-substituted toluene derivative of the general formula (1).

(Ethereal Solvent)

The ethereal solvent usable in the Grignard reaction of the invention includes, for example, cyclic ethers such as tetrahydrofuran, tetrahydropyran, tetrahydrofurfuryl ethyl ether, 2-ethoxytetrahydropyran, and dihydropyran; and chain ethers such as diethyl ether, diisopropyl ether, dibutyl ether, 1,2-dimethoxyethane, and diethylene glycol dimethyl ether.

In terms of reactivity, the use of the cyclic ether is preferred. If the ease of industrial acquisition is also considered, the cyclic ether, such as tetrahydrofuran or tetrahydropyran, can be used particularly preferably.

Any of these ethereal solvents is preferably minimal in water content in order to suppress a side reaction. Preferably usable is the ethereal solvent in which the proportion of water (weight = B) contained therein based on pure ether (weight = A) (i.e., water content = B/A ) is 100 ppm or lower (further, 50 ppm or lower). To measure such a water content, the use of the Karl Fischer's method or the like is sufficient.

In the invention, one or more hydrocarborr solvents (boiling point, etc. are not restricted) are suitably mixed, where necessary, with any such ethereal solvent, whereby the use of a relatively expensive ethereal solvent can be decreased. Examples of the hydrocarbon solvent usable for this purpose are aromatic hydrocarbons such as benzene, toluene, xylene and mesitylene; and aliphatic or alicyclic hydrocarbons such as hexane, cyclohexane, methylcyclohexane, and ligroin.

In view of easy acquisition, reactivity, and manufacturing cost, the aromatic hydrocarbons, especially, toluene and/or xylene can be used preferably. In terms of the reactivity in the Grignard reaction, however, it is preferred not to make the proportion of the ethereal solvent excessively low. Normally, the mixing ratio of the hydrocarbon solvent is preferably 10:1 or less (further, 5:1 or less) to the ethereal solvent when expressed in the volume ratio. To reduce the cost of the solvents, on the other hand, the mixing ratio of the hydrocarbon solvent is preferably 1:1 (further, 1.2:1 or more) to the ethereal solvent when expressed in the volume ratio.

The amount of the ethereal solvent (or a mixture of the ethereal solvent and the hydrocarbon solvent; the same will hold in the descriptions to follow) is preferably 0.2 to 4 liters (further, 0.3 to 2 liters) per mol of the 2-chloro-6-substituted toluene derivative of the general formula (1).

(one Embodiment of Reaction Procedure)

A preferred embodiment of the reaction procedure for the Grignard reaction of the invention will be described.

In this embodiment, it is preferred to charge metallic magnesium into an ethereal solvent, then stir the mixture, and add a lower alkyl bromide dropwise to form a lower alkylmagnesium brpmide in the solvent.

(Formation-of Lower Alkylmagnesium Bromide)

As noted above, it is preferred in the invention that a lower alkyl bromide is reacted with metallic magnesium in a reaction system to form a lower alkylmagnesium bromide in the system.

The amount of the lower alkyl bromide used is preferably in the range of from 0.05 to 1 mol (further, in the range of from 0.1 to 0.5 mol) per mol of the 2-chloro-6-substituted toluene derivative of the general formula (1). The reaction temperature for the formation of the lower alkylmagnesium bromide is preferably 10 to 100°C (further, 30 to 50°C), and may be controlled to this range, for example, by cooling the reaction system as desired.

(Reaction with 2-Chloro-6-Substituted Toluene Derivative)

Then, the 2-chloro-6-substituted toluene derivative of the general formula (1) is added into the resulting lower alkyl bromide/ether, whereby the derivative is reacted with metallic magnesium in the copresence of the lower alkylmagnesium bromide to form a 2-methylphenylmagnesium halide (2) having a substituent at the 3-position (Grignard reagent).

This is preferred, because the lower alkylmagnesium bromide has been formed previously in the system, and then the 2-chloro-6-substituted toluene derivative (1) is added to the reaction system, whereby the 2-methylphenylmagnesium halide (2) having a substituent at the 3-position can be formed, with a reaction promoting effect of the lower alkylmagnesium bromide being imparted.

Upon completion of the reaction, the lower alkylmagnesium bromide present beforehand in the system and the 2-methylphenylmagnesium halide (2) having a substituent at the 3-position are normally coexistent.

The reaction temperature for the reaction forming the 2-methylphenylmagnesium halide (2) having a substituent at the 3-position is preferably in the range of from 50 to 140°C (further, 60 to 120°C) in order to maintain an appropriate reaction rate and prevent a side reaction.

Whether the reaction has proceeded fully and the 2-methylphenylmagnesium halide (2) having a substituent at the 3-position has been formed can be determined, for example, by confirming the disappearance of the 2-chloro-6-substituted toluene derivative (1) by gas chromatography or the like. The preferred reaction time is normally about 2 to 15 hours (further, 3 to 10 hours).

(Reaction with Carbon Dioxide)

After the reaction is confirmed to have proceeded sufficiently, carbon dioxide is reacted with the resulting 2-methylphenylmagnesium halide (2), where necessary. More specifically, the reaction mixture containing the 2-methylphenylmagnesium halide (2) is cooled, and a carbon dioxide gas is blown into the reaction mixture, or dry ice (solid carbon dioxide) is charged into the reaction mixture to add carbon dioxide. The amount of carbon dioxide used is preferably 1 mol or more (further, 1 to 20 mols) per mol of the 2-chloro-6-substituted toluene derivative of the general formula (1).

As described above, in the invention, carbon dioxide is successively reacted with the resulting Grignard reagent (2), where necessary. Since this reaction is a strong exothermic reaction, it is preferred to cool the reaction system thoroughly. More concretely, the reaction temperature is preferably -10 to 50°C (further, 0 to 40°C). Completion of the reaction can be confirmed by the fact that heat generation by the introduction of carbon dioxide has been completed, or by the knowledge of the amount of carboxylic acid formation based on gas chromatography or high performance liquid chromatography after treatment of the reaction mixture with water and an acid. The preferred reaction time is normally 1 to 10 hours (further, 2 to 7 hours).

(Reaction with Acid)

After the foregoing reaction, an acid is added, where necessary, whereby an o-toluic acid of the general formula (4) can be obtained in a high yield:

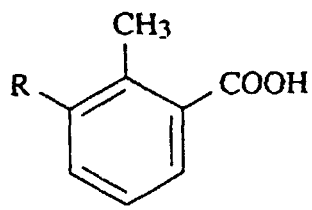

···(4)

wherein R denotes a fluorine atom, a chlorine atom, an alkoxy group, or an alkylthio group.

In the invention, after reaction with carbon dioxide, treatment with an acid is performed, where necessary, in order to liberate a carboxyl group introduced into the phenyl ring. The acid used here is not restricted, as long as it is an acid capable of releasing a proton. However, the use of hydrochloric acid or sulfuric acid is preferred, because of the ease of handling and economy. This acid treatment makes it possible to isolate the desired product (4) in the form of a free carboxylic acid.

(Reaction Equipment)

The Grignard reaction of the invention can be performed using ordinary agitation type reaction equipment. To prevent the decomposition of the Grignard reagent due to incorporation of moisture or oxygen, however, it is preferred that the reaction be performed in a stream of nitrogen or in a closed system.

The present invention will be described in greater detail by way of Examples.

Examples

<u>Example 1</u>

(Synthesis of 2-chloro-6-methoxytoluene)

A 300 mL tour-necked flask equipped with a Dimroth reflux condenser, a thermometer, a rectification column (size: 30 cm x 1.8 cm in diameter, packed with about 50 g of glass Raschig rings with an average diameter of 3 mm), and a stirrer was charged with 32.2 g (0.2 mol) of 2,6-dichlorotoluene, 57.8 g (0.3 mol) of sodium methylate (a 28% methanol solution; a synthetic product from metallic sodium and methanol was used unchanged), and 140 mL of dimethyl sulfoxide. The system was stirred on an oil bath, and continuously heated with stirring until the liquid temperature reached 120°C, when methanol began to be distilled off from the top of the column. Heating with stirring was continued in this state for about 30 minutes, and when distilling off of methanol was nearly complete, the temperature of the reaction mixture reached 150°C. Then, the system was heated for a further 3.5 hours at the reaction mixture temperature of 150 to 155°C.

About 0.2 mL of the resulting reaction mixture was sampled with a dropping pipette, and acid treated (about 1 mL of 10% hydrochloric acid was added). Analysis of the sample by GC (gas chromatography) showed that 2-chloro-6-methoxytoluene was formed in a proportion of 50%, and 2-chloro-6-hydroxytoluene was formed in a proportion of 48%, based on the total area of the GC peaks. The conditions for GC were as follows:

〈GC conditions〉

GC device: product of Shimadzu Corp., trade name: GC-9A
Column: product of Kabushiki Kaisha Kagakihin Kensa Kyokai, trade name: G-100, length: 40 m, internal diameter 1.2 mm, film thickness 1 μm
Column oven temperature: 80°C (kept for 3 minutes) to 250°C (heated at a rate of 10°C/min)
Detector: FID
Recorder: product of Shimadzu Corp., trade name: C-R6A

The resulting reaction mixture was cooled to 95°C, and 3.9 g (0.02 mol) of sodium methylate (28% methanol solution) was added dropwise. Then, 13.9 g (0.11 mol) of dimethylsulfuric acid was added dropwise, whereupon heat was generated to raise the liquid temperature to 105°C. Then, the system was slightly cooled, and stirred for 30 minutes at 95°C.

Then, the reaction mixture was cooled to room temperature, whereafter inorganic salts that precipitated were removed by filtration. Quantitative analysis of the filtrate by GC showed that 2-chloro-6-methoxytoluene was formed in a proportion of 92% (based on 2,6-dichlorotoluene). The GC conditions employed on this occasion were the same as those mentioned above.

The resulting filtrate was extracted three times with 200 mL of cyclohexane. The resulting cyclohexane phase was washed with 500 mL of water, and then allowed to stand overnight over anhydrous sodium sulfate at room temperature for drying. Then, cyclohexane was distilled off to obtain 28.5 g of 2-chloro-6-methoxytoluene.

The physical properties (boiling point = 94 to 97°C /14 mmHg) and spectrum data (IR and NMR) of the resulting product agreed with the substance synthesized based on the method described in Japanese Unexamined Patent Publication No. 56-170780 (Referential Example 5). The yield of the product was 91.0% (based on 2,6-dichlorotoluene), and its purity was 97.5% (determined by the gas chromatography total area method).

Example 2

A 2-liter four-necked flask equipped with a reflux condenser, a thermometer, a rectification column (packed with glass Raschig rings), and a stirrer was charged with 161.0 g (1.0 mol) of 2,6-dichlorotoluene, 289.4 g (1.5 mol) of sodium methylate (a 28% methanol solution), and 700 mL (770 g) of dimethyl sulfoxide. The system was heated on an oil bath over 2 hours, with stirring, until the liquid temperature reached 150°C. During this period, 152.3 g of methanol was recovered from the rectification column. Further, heating with stirring was continued for 7 hours at 150°C.

The reaction mixture was sampled, acid treated, and then analyzed by GC. 2-Chloro-6-methoxytoluene was formed in a proportion of 52%, and 2-chloro-6-hydroxytoluene was formed in a proportion of 45%, based on the total area.

The resulting reaction-mixture-was cooled to room temperature, and 63.1 g (0.5 mol) of dimethylsulfuric acid was added dropwise. As a result, heat was generated to raise the liquid temperature to 45°C. Then, the system was heated, with stirring, for 1 hour at 60°C. After completion of the reaction, the reaction mixture was cooled to room temperature, and inorganic salts that precipitated were removed by filtration. The filtrate was extracted three times with 500 mL of cyclohexane. The resulting cyclohexane phase was washed with 500 mL of a 2% aqueous solution of sodium hydroxide, and further washed twice with 500 mL of water. Then, cyclohexane was refluxed under heat, and water was removed by a water separator, followed by distilling off cyclohexane. Then, the residue was distilled under reduced pressure to obtain 124.0 g of 2-chloro-6-methoxytoluene (boiling point 72°C/5 mmHg) in a yield of 79.2%.

The dimethyl sulfoxide layer that remained during the above extraction was distilled under reduced pressure to recover 700.4 g of dimethyl sulfoxide. The recovered solvent contained 3.8% by weight of 2-chloro-6-methoxytoluene (corresponding to a yield of 14.6%). The recovery ratio of dimethyl sulfoxide was 88.0%.

Example 3

A 1-liter four-necked flask equipped with a reflux condenser, a thermometer, a rectification column (packed with glass-Raschig rings), and a stirrer was charged with 80.5 g (0.5 mol) of 2,6-dichlorotoluene, 144.7 g (0.75 mol) of sodium methylate (a 28% methanol solution), and 350 mL of 1,3-dimethyl-2-imidazolidinone. The system was heated on an oil bath over 1.5 hours, with stirring, until the liquid temperature reached 150°C. Simultaneously, methanol was

recovered. Further, heating with stirring was continued for 6 hours at 150°C.

The reaction mixture was sampled, acid treated, and then analyzed by GC. 2-Chloro-6-methoxytoluene was formed in a proportion of 56%, and 2-chloro-6-hydroxytoluene was formed in a proportion of 42%, based on the total area.

The resulting reaction mixture was cooled to 30°C, and 31.5 g (0.25 mol) of dimethylsulfuric acid was added dropwise. As a result, heat was generated to raise the liquid temperature to 50°C. Then, the system was heated, with stirring, for 3 hours at 65°C. After completion of the reaction, the reaction mixture was cooled to room temperature, 700 mL of water was added, and the mixture was extracted twice with 200 mL of cyclohexane. The resulting cyclohexane phase was washed with 500 mL of a 2% aqueous solution of sodium hydroxide, and further washed twice with 400 mL of water. The cyclohexane layer was withdrawn, and dried over anhydrous sodium sulfate. Then, cyclohexane was distilled off to obtain 72.1 g (yield 92.1%) of 2-chloro- 6-methoxytoluene.

Examples 4 to 5

The following reaction was performed in the same manner as in Example 3, except that the aprotic polar solvent was changed:

The reaction scale was 0.1 mol. 2,6-Dichlorotoluene and a methanol solution of sodium methylate were reacted for a predetermined time at 150°C, with methanol being distilled off, and then the system was cooled to 90°C. At the same temperature, dimethylsulfuric acid was added, whereupon the temperature of the reaction mixture temporarily rose to 100°C. Then, the reaction was performed for 1 hour at 90°C, and after completion of the reaction, inorganic matter was removed by filtration. The resulting product in the filtrate was quantitatively analyzed by GC using the internal standard method (internal standard: 4,4-dimethyldiphenyl ether). The results are shown in Table 1.

Table 1

| | Aprotic polar solvent 1) | Reaction temp. (°C)/reaction time (hr) | 2-Chloro-6-methoxytoluene formation (%) |
|---|---|---|---|
| Ex. 4 | DMI | 150/5 | 89.2 |
| Ex. 5 | $TMSO_2$ | 150/18.5 | 66.8 |

1) DMI = 1,3-Dimethyl-2-imidazolidinone
$TMSO_2$ = Sulfolane

Example 6

A 300 mL four-necked flask equipped with a reflux condenser, a thermometer, a tectification column (packed with glass Raschig rings), and a stirrer was charged with 16.1 g (0.1 mol) of 2,6-dichlorotoluene, 51.0 g (0.15 mol) of sodium ethylate (a 20% ethanol solution), and 70 mL of dimethyl sulfoxide. The system was heated to 125°C on an oil bath, with stirring, and gradually heated to 150°C over 30 minutes. During this period, 29.5 g of ethanol was recovered. Then, stirring was continued for 5 hours at the same temperature.

The resulting reaction mixture was sampled, acid treated, and then analyzed by GC. 2-Chloro-6-ethoxytoluene was formed in a proportion of 62.7%, and 2-chloro-6-hydroxytoluene was formed in a proportion of 32.1%, based on the total area.

The resulting reaction mixture was cooled to room temperature, 1.2 g (0.02 mol) of 95% potassium hydroxide was added, and then 6.5 g (0.06 mol) of ethyl bromide was added dropwise. Then, the system was heated to 85°C, and heated, with stirring, for 3 hours at the same temperature. After completion of the reaction, the reaction mixture was cooled to room temperature, and inorganic salts that precipitated were removed by filtration. The filtrate was extracted three times with 100 mL of n-hexane. The resulting n-hexane phase was washed with 200 mL of water, and then dried over anhydrous sodium sulfate. Then, n-hexane was distilled off, and finally the residue was distilled under reduced pressure to obtain 16.0 g (yield 92.1%) of 2-chloro-6-ethoxytoluene (purity 95.6%, boiling point 82-85°C/3 mmHg).

Example 7

A 200 mL four-necked flask equipped with a reflux condenser, a thermometer, and a stirrer was charged with 50mL of isopropanol, and 3.45g (0.15 mol) of metallic sodium pieces was gradually charged into the flask. Then, 50 mL of isopropanol was further added, and the system was heated to 75°C, followed by heating with stirring for 1 hour at the same temperature. Complete dissolution of the metallic sodium was confirmed, and slightly air cooled. Then, a distillation device was equipped on the flask, and the system was reheated to recover 57 mL of isopropanol at atmospheric pressure. After the reaction mixture (the alcohol solution of the metal alcoholate) was cooled, a rectification column was mounted on the distillation device.

Then, the four-necked flask containing the alcohol solution of the metal alcoholate was charged with 16.1 g (0.1 mol) of 2,6-dichlorotoluene and 70 mL of dimethyl sulfoxide. The system was heated over 30 minutes on an oil bath, with stirring, until it reached 150°C. At the same time, 15.0 g of isopropanol was recovered from the rectification column. Further, heating with stirring was continued for 6 hours at 150°C.

The resulting reaction mixture was sampled, acid treated, and then analyzed by GC. 2-Isopropoxy-6-chlorotoluene was formed in a proportion of 43.5%, 2-chloro-6-hydroxytoluene was formed in a proportion of 27.6%, and 2,6-dichlorotoluene remained in a proportion of 10.4%, based on the total area.

The resulting reaction mixture was allowed to stand overnight, and then 5.1 g (0.041 mol) of isopropyl bromide was added dropwise. Then, the system was heated to 100°C, and heated, with stirring, for 2 hours at the same temperature. The composition (the proportions based on the total area of the GC peaks) of the reaction mixture was: 7.8% of 2,6-dichlorotoluene, 67.6% of 2-isopropoxy-6-chlorotoluene, and 5.7% of 2-chloro-6-hydroxytoluene.

To the reaction mixture, 1.0g (0.017 mol) of 95% potassium hydroxide and 2.4 g (0.02 mol) of isopropyl bromide were further added. When the mixture was stirred for 3 hours at 100°C, the residue of 2-chloro-6-hydroxytoluene was 0.2%.

After the reaction mixture was cooled, the same posttreatment as in Example 6 was performed. The resulting crude product was distilled to obtain 13.1 g (yield 70.9%) of 2-isopropoxy-6-chlorotoluene (purity 84.2%, boiling point 81-84°C/3 mmHg).

<u>Comparative Example 1</u>

A 50 mL four-necked flask equipped with a reflux condenser, a thermometer, and a stirrer was charged with 2.42 g (0.015 mol) of 2,6-dichlorotoluene, 1.62 g (0.030 mol) of sodium methoxide powder, and 15 mL of 1,3-dimethyl-2-imidazolidinone. The system was heated to 160°C on an oil bath, with stirring, and stirred for 3 hours at the same temperature. The composition (the proportions based on the total area of the GC peaks) of the reaction mixture was: 84% of 2,6-dichlorotoluene, 15% of 2-chloro-6-methoxytoluene, and 0.8% of 2-chloro-6-hydroxytoluene.

<u>Example 8</u>

(Grignard Reaction)

A 1-liter four-necked flask equipped with a reflux condenser, a thermometer, and a stirrer was charged with 180 mL of anhydrous tetrahydrofuran, 20.4 g (0.84 mol) of metallic magnesium powder, and 240 mL of xylene. Under a nitrogen current, 22.1 g (0.18 mol) of isopropyl bromide was gradually added dropwise, with stirring, over 50 minutes at 40 to 43°C. After completion of dropwise addition, the system was heated on an oil bath, and continued to be stirred for 2 hours at 40°C. Then, 94.0 g (0.6 mol) of 2-chloro-6-methoxytoluene was added dropwise over 10 minutes, whereafter the system was heated on an oil bath, and stirred for 8 hours at 90 to 95°C.

The reaction mixture was cooled with ice to 3°C, and carbon dioxide was blown into the reaction mixture, whereupon heat generation began. With the system being cooled with iced water, carbon dioxide continued to be blown into it for about 1 hour at 10 to 16°C. After heat generation was completed, cooling was terminated, and carbon dioxide was blown into the reaction mixture for a further 30 minutes at 15 to 18°C. The total amount of carbon dioxide blown into the reaction mixture was 36.2 g (0.82 mol).

0.2 mL of the resulting reaction mixture was sampled, and treated with water and an acid (1 mL of 10% hydrochloric acid was added). Analysis of the sample by GC showed that 2-chloro-6-methoxytoluene as the starting material was present in a proportion of 1.9%, 2-methoxytoluene probably resulting from dechlorination of the starting material because of Grignard reagent formation was present in a proportion of 1.2%, and 2-methyl-3-methoxybenzoic acid as the desired product was formed in a proportion of 92.4%, each percentage based on the total area. The conditions for GC were as follows:

〈GC conditions〉

GC device: product of Shimadzu Corp., trade name: GC-9A
Column: product of Kabushiki Kaisha Kagakuhin Kensa Kyokai, trade name: G-100, length: 40 m, internal diameter 1.2 mm, film thickness 1 μm

Column oven temperature: 80°C (kept for 3 minutes) to 250°C (heated at a rate of 10°C/min)
Detector: FID
Recorder: product of Shimadzu Corp., trade name: C-R6A

After blowing-in of carbon dioxide was completed, 300 mL of water was added to the reaction mixture dropwise at 20 to 35°C, with stirring, while the reactor was being cooled with water. Then, 117.7 g of 60% sulfuric acid was added dropwise, and the mixture was stirred for 30 minutes. When the reaction mixture was heated to 60°C, it was separated into two phases.

This reaction mixture separated into two phases was transferred into a separating funnel. The organic phase was separated, and alkali-extracted with a mixture of 500 mL of water and 126 g of 48% sodium hydroxide. After phase separation, the aqueous phase was withdrawn, and washed with 300 mL of xylene. After further phase separation, the resulting aqueous phase was withdrawn, and 141.2 g of 60% sulfuric acid was added dropwise, with stirring, to make the phase acidic. Then, the aqueous phase was cooled to room temperature, whereafter precipitated crystals were collected by filtration. The crystals were washed with water, and then dried to obtain 89.6 g of 2-methyl-3-methoxybenzoic acid (yield 90%).

Example 9

A 300 mL four-necked flask equipped with a reflux condenser, a thermometer, and a stirrer was charged with 70 mL of anhydrous tetrahydrofuran, and 3.89 g (0.16 mol) of metallic magnesium powder. Under a nitrogen current, 6.15 g (0.05 mol) of isopropyl bromide was gradualy added dropwise, with stirring, over 10 minutes at 26 to 47°C. After completion of dropwise addition, stirring was continued for 25 minutes at room temperature.

Then, 15.7 g (0.1 mol) of 2-chloro-6-methoxytoluene was added dropwise over 5 minutes at room temperature, whereafter the system was heated on an oil bath, and stirred for 9 hours at 72°C. The reaction mixture was cooled with ice to 5°C, and 40 g (0.91 mol) of crushed dry ice pieces were successively thrown into the reaction mixture over 20 minutes. As a result, heat generation occurred to raise the liquid temperature to 30°C. Then, the reaction system was closed with a gas balloon, and stirred for 1 hour at room temperature.

Then, 50 mL of water was added to the reaction mixture dropwise at 20 to 35°C, with stirring, while the reactor was being cooled with water. Then, 35 mL of 35% hydrochloric acid was added dropwise, and the mixture was stirred for 30 minutes. When the reaction mixture was further heated to 60°C, it was separated into two phases. This reaction mixture separated into two phases was transferred into a separating funnel. The organic phase was separated, and alkali-extracted with a mixture of 200 mL of water and 18 g of 48% sodium hydroxide. After phase separation, the aqueous phase was withdrawn, and washed with 100 mL of toluene. After further phase separation, the resulting aqueous phase was withdrawn; and 35 mL of 35% hydrochloric acid was added dropwise, with stirring, to make the phase acidic. Then, the aqueous phase was cooled to room temperature, whereafter precipitated crystals were collected by filtration. The crystals were washed with water, and then dried to obtain 15.4 g of 2-methyl-3-methoxybenzoic acid (yield 93%).

Example 10

A 300 mL four-necked flask equipped with a reflux condenser, a thermometer, and a stirrer was charged with 30 mL of anhydrous tetrahydrofuran, 3.16 g (0.13 mol) of metallic magnesium powder, and 40 mL of xylene. Under a nitrogen current, 2.46 g (0.02 mol) of isopropyl bromide was gradually added dropwise, with stirring, over 10 minutes at room temperature to 44°C. After completion of dropwise addition, stirring was continued for 1 hour at room temperature.

Then, 15.4 g (0.1 mol) of 2-chloro-6-fluorotoluene was added dropwise over 5 minutes at room temperature, whereafter the system was heated on an oil bath, and stirred for 4 hours at 90°C. The reaction mixture was cooled with ice to 3°C, and in the cooled condition, dry ice was thrown into the reaction mixture over 30 minutes at 35°C or lower until heat generation ended. The total amount of dry ice thrown into the reaction mixture was 40 g (0.91 mol). Then, the reaction system was closed with a gas balloon, and stirred for 2 hours at room temperature. Then, 50 mL of water was added to the reaction mixture dropwise at 40°C or lower, with stirring, while the reactor was being cooled with water. Then, 25 mL of 35% hydrochloric acid was added dropwise, and the mixture was stirred for 30 minutes.

When the reaction mixture was further heated to 60°C, it was separated into two phases. This reaction mixture was transferred into a separating funnel. The organic phase was separated, and alkali-extracted with a mixture of 100 mL of water and 21 g of 48% sodium hydroxide. After phase separation, the aqueous phase was withdrawn, and 33 mL of 35% hydrochloric acid was added dropwise to make the phase acidic. Then, the aqueous phase was cooled to room temperature, whereafter precipitated crystals were collected by filtration. The crystals were washed with water, and then dried to obtain 13.9 g of 3-fluoro-2-methylbenzoic acid (yield 90%).

Example 11

A 200 mL four-necked flask equipped with a reflux condenser, a thermometer, and a stirrer was charged with 60 mL of anhydrous tetrahydrofuran and 3.40 g (0.14 mol) of metallic magnesium powder. Under a nitrogen current, 3.27 g (0.03 mol) of ethyl bromide was gradually added dropwise, with stirring, over 5 minutes at 28 to 55°C. After completion of dropwise addition, stirring was continued for 30 minutes at room temperature. Then, 15.7 g (0.1 mol)of 2-chloro-6-methoxytoluene was added dropwise over 5 minutes at room temperature, whereafter the system was heated on an oil bath, and stirred for 9 hours at 70°C.

The resulting reaction mixture was cooled with ice to 3°C, and in the cooled condition, dry ice was thrown into the reaction mixture over about 1 hour at room temperature to 20°C until heat generation ended. The total amount of dry ice thrown into the reaction mixture was 51 g (1.16 mol). After the charging of dry ice was completed, 50 mL of water was added dropwise at 20 to 35°C, with stirring, while the reactor was being cooled with water. Then, 25 mL of 35% hydrochloric acid was added dropwise, and then the mixture was stirred for 30 minutes. When the reaction mixture was further heated to 60°C, it was separated into two phases.

This reaction mixture was transferred into a separating funnel. The organic phase was separated, and alkali-extracted with a mixture of 100 mL of water and 21 g of 48% sodium hydroxide. After phase separation, the aqueous phase was withdrawn, and washed with 50 mL of xylene. After further phase separation, the aqueous phase was withdrawn, and 33 ml of 35% sulfuric acid was added dropwise, with stirring, to make the phase acidic. Then, the mixture was cooled to room temperature, whereafter precipitated crystals were collected by filtration. The crystals were washed with water, and then dried to obtain 9.46 g of 2-methyl-3-methoxybenzoic acid (yield 57%).

Comparative Example 2

(Follow-up of Japanese Unexamined Patent Publication No. 58-17080)

A follow-up was carried out in accordance with the Referential Example 6 of Japanese Unexamined Patent Publication No. 58-17080.

A solution of 0.03 mol of 2-methoxy-6-chlorotoluene in anhydrous tetrahydrofuran (0.83 L/mol) was refluxed for 24 hours at 70°C under a nitrogen current together with Mg (1.4 equivalents) and iodine ($I_2$ in a trace amount, a piece of $I_2$ with a major diameter of about 2 mm; Japanese Unexamined Patent Publication No. 58-17080 does not describe the amount of iodine used).

The resulting solution was cooled, and carbon dioxide was blown into the solution for 2 hours. Then, the solution was evaporated, and the residue was dissolved in 10% sodium carbonate. The solution was extracted with ethyl acetate, but no reaction had occurred. That is, the desired o-toluic acid was not detected under the aforementioned GC conditions, and only the starting material (2-methoxy-6-chlorotoluene) was recovered from the ethyl acetate extract.

Comparative Example 3

The reaction was attempted in the same manner as in Comparative Example 2, except that the amount of 2-methoxy-6-chlorotoluena was 0.2 mol (about 7 times the amount in Comparative Example 2) and the amount of iodine was 0.3 g/mol (a large excess amount compared with the amount normally used).

Four hours after initiation of the reaction, no reaction had occurred as in the case of Comparative Example 2. However, a continued reaction (24 hours after initiation of the reaction) obtained a predetermined Grignard reagent (yield by GC analysis: 78.9%). After reaction of the Grignard reagent with carbon dioxide, 3-methoxy-2-methylbenzoic acid was present (yield 76%).

The results obtained in Comparative Examples 2 to 3 are summarized in Table 2.

Table 2

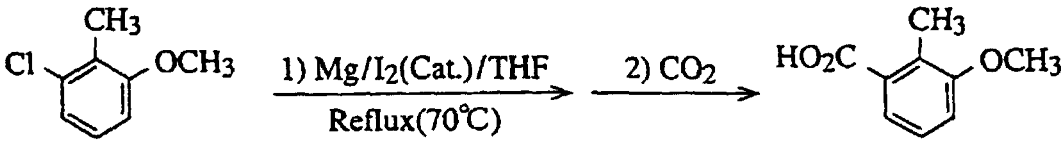


| | Scale (mol) | Mg (eq) | $I_2$ (g/mol) | Solvent (1/mol) | Time (h) | Yield (%) |
|---|---|---|---|---|---|---|
| Comp.Ex.2 | 0.03 | 1.4 | Trace | THF (0.83) | 24 | No reaction |
| Comp.Ex.3 | 0.2 | 1.4 | 0.3 | THF (0.83) | 4<br>↓<br>24 | No reaction<br><br>GC 78.9% (Grignard)<br>Isolated after reaction with $CO_2$: 77.5% |
| Jap.Unex. Pat.Pub. 58-170780 | 0.036 | 1.42 | Not described | 0.824 | 24 | Isolated after reaction with $CO_2$: 76% |

As shown in Table 2, the iodine catalyst method of Comparative Examples 2 to 3 was problematical in terms of reproducibility in comparison with the Grignard reaction of the present invention (alkyl bromide addition method). Comparative Example 3, in particular, has a very long induction period of 4 hours after initiation of the reaction, and thus is at least unsuitable for industrial use. The cause of these phenomena is presumed, according to the inventors' findings, to be as follows: With the iodine catalyst method, the difficulty or ease of initiation of reaction, in particular, depends greatly on the surface state of metallic magnesium. Thus, the reactivity varies greatly according to the variation in the quality of the metallic magnesium, or according to whether the metallic magnesium is old or new.

In the present invention, on the other hand, the following mechanism is presumed to work: The alkylmagnesium bromide with high reactivity is made existent in the system. At the start of the reaction, therefore, the surface of metallic magnesium has been easily activated because of constant contact between the alkylmagnesium bromide and the metallic magnesium. Furthermore, the Grignard reaction of an aromatic chlorine compound (generally considered to be inert) can proceed smoothly through a catalytic Grignard exchange reaction ascribed to the coexistent alkylmagnesium halide.

**Industrial Applicability**

As described above, the present invention provides a process for preparing a toluene derivative of the following general formula (2)

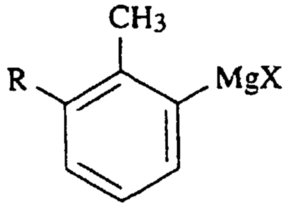

···(2)

wherein R denotes a fluorine atom, a chlorine atom, an alkoxy group, or an alkylthio group, and X denotes a halogen

atom, comprising reacting metallic magnesium with a 2-chloro-6-substituted toluene derivative of the general formula (1)

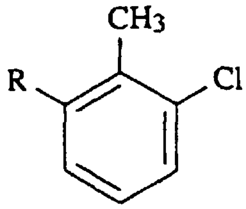

···(1)

wherein R is as defined above, in an ethereal solvent in the presence of a lower alkyl bromide.

The invention also provides a process for preparing a toluene derivative of the general formula (3)

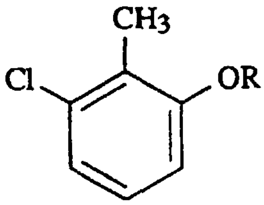

···(3)

wherein R denotes a lower alkyl group, comprising reacting 2,6-dichlorotoluene and an alcohol solution of a metal alcoholate in an aprotic polar solvent, while distilling off the alcohol, and then reacting an alkylating agent with the resulting product.

According to the present invention of the foregoing constitution, a Grignard reagent can be obtained with high reproducibility and in a high yield from the corresponding 2-chloro-6-sustituted toluene, an aromatic chlorine compound thus far regarded as being inert. The resulting Grignard reagent is very useful as an active synthetic intermediate for the production of various physiologically active substances (e.g., pharmaceuticals and agrochemicals). For example, the invention provides a preferred process for the industrial production of an o-toluic acid having a substituent at the 3-position, by reacting the Grignard reagent with carbon dioxide.

Furthermore, the invention provides a process for preparing a 2-alkoxy-6-chlorotoluene, a useful starting material for the Grignard reaction, in a satisfactory yield without absolutely necessitating the use of a solvent which is toxic and which poses difficulties in industrial use.

**Claims**

**1.** A process for preparing a toluene derivative of the general formula (2)

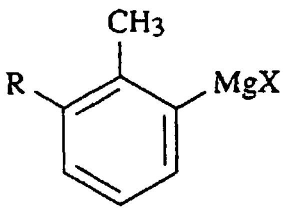

···(2)

wherein R denotes a fluorine atom, a chlorine atom, an alkoxy group, or an alkylthio group, and X denotes a halogen atom, comprising reacting metallic magnesium with a 2-chloro-6-substituted toluene derivative of the general formula (1)

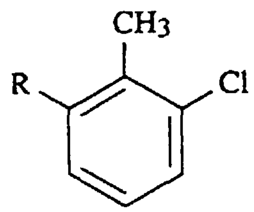

...(1)

wherein R is as defined above, in an ethereal solvent in the presence of a lower alkyl bromide.

2. A process for preparing a toluene derivative according to claim 1, wherein the lower alkyl bromide is isopropylmagnesium bromide.

3. A process for preparing a toluene derivative of the general formula (4)

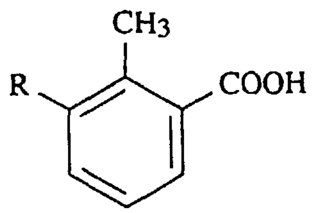

...(4)

wherein R is as defined above, comprising further reacting carbon dioxide with the toluene derivative (2) according to claim 1.

4. A process for preparing a toluene derivative of the general formula (3)

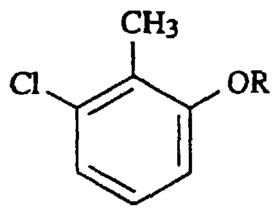

...(3)

wherein R denotes a lower alkyl group, comprising reacting 2,6-dichlorotoluene and an alcohol solution of a metal alcoholate in an aprotic polar solvent, while distilling off the alcohol, and then reacting an alkylating agent with the resulting product.

5. A process for preparing a toluene derivative according to claim 4, wherein the aprotic polar solvent is dimethyl sulfoxide or 1,3-dimethyl-2-imidazolidinone.

6. A process for preparing a toluene derivative according to claim 4 or 5, wherein the alkylating agent is reacted in the presence of a base.

7. A process for preparing a toluene derivative according to claim 5, wherein the aprotic polar solvent is dimethyl sulfoxide, and the desired product is extracted with an aliphatic or alicyclic hydrocarbon solvent, with no water being added to a reaction mixture obtained upon completion of the reaction.

# INTERNATIONAL SEARCH REPORT

International application No. PCT/JP98/05571

A. CLASSIFICATION OF SUBJECT MATTER
Int.Cl$^6$ C07F3/02, C07C41/06 , C07C43/225, C07C63/04, C07C65/21, C07C51/15, C07C323/62, C07C319/20
According to International Patent Classification (IPC) or to both national classification and IPC

B. FIELDS SEARCHED
Minimum documentation searched (classification system followed by classification symbols)
Int.Cl$^6$ C07F3/02, C07C41/06K, C07C43/225, C07C63/04, C07C65/21, C07C51/15, C07C323/62, C07C319/20

Documentation searched other than minimum documentation to the extent that such documents are included in the fields searched

Electronic data base consulted during the international search (name of data base and, where practicable, search terms used)
CAPLUS (STN), CAOLD (STN), REGISTRY (STN)

C. DOCUMENTS CONSIDERED TO BE RELEVANT

| Category* | Citation of document, with indication, where appropriate, of the relevant passages | Relevant to claim No. |
|---|---|---|
| PX | IKOMA, Yoshiharu; NAOI, Yoshitake, "Industrial synthesis of 3-chloro-2-methylbiphenyl by non-ligated nickel (II) chloride-catalyzed cross-coupling of aryl Grignard reagents with haloarenes", Nippon Kagakkaishi (1998), (7), p.514-517 | 1-2 |
| X | LAI, Yee-Hing; CHEN, Pu; DINGLE, Thomas W., "Synthesis and Diatropicity of trans-10b, 10c-Dimethylacenaphthyleno[1,2:e]-10b, 10c-dihydropyrene : A Model Aromatic Molecule To Verify the Effect of conjugation on the Diatropicity of an Annulene", J. Org. Chem. (1997), 62(4), p.916-924 | 1-2 |
| X | JP, 63-295520, A (Yuki Gosei Kogyo Co., Ltd.), 1 December, 1988 (01. 12. 88) (Family: none) | 1-2 |

[X] Further documents are listed in the continuation of Box C. [ ] See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 19 February, 1999 (19. 02. 99) | 2 March, 1999 (02. 03. 99) |
| Name and mailing address of the ISA/ Japanese Patent Office | Authorized officer |
| Facsimile No. | Telephone No. |

Form PCT/ISA/210 (second sheet) (July 1992)

INTERNATIONAL SEARCH REPORT

International application No. PCT/JP98/05571

C (Continuation). DOCUMENTS CONSIDERED TO BE RELEVANT

| Category* | Citation of document, with indication, where appropriate, of the relevant passages | Relevant to claim No. |
|---|---|---|
| Y | MILLER, William H.; KU, Thomas W.; ALI, Fadia E.; BONDINELL, William E.; CALVO, Raul R.; DAVIS, Larry D.; ERHARD, Karl F.; HALL, Leon B.; HUFFMAN, William F.; et al., "Enantiospecific synthesis of SB 214857, a potent, orally active, nonpeptide fibrinogen receptor antagonist", Tetrahedron Lett. (1995), 36(52), p.9433-9436 | 3 |
| Y | JP, 9-508620, A (SmithKline Beecham Corp.), 2 September, 1997 (02. 09. 97) & WO, 95/18619, A1 & EP, 738150, A1 | 3 |
| Y | BENNETAU, Bernard; MORTIER, Jacques; MOYROUD, Joeel; GUESNET, Jean-Luc, "Directed lithiation of unprotected benzoic acids", J. Chem. Soc., Perkin Trans. 1 (1995), (10), p.1265-1271 | 3 |
| Y | DE, 2527321, A1 (Merck and Co., Inc.), 23 December, 1976 (23. 12. 76) (Family: none) | 3 |
| PX | JP, 10-195017, A (Rohm and Haas Co.), 28 July, 1998 (28. 07. 98) (Family: none) | 4-7 |
| Y | TESTAFERRI, L.; TIECCO, M.; TINGOLI, M.; CHIANELLI, D.; MONTANUCCI, M., "The reactions of unactivated aryl halides with sodium methoxide in HMPA. Synthesis of phenols, anisoles, and methoxyphenols", Tetrahedron (1983), 39(1), p.193-197 | 4-7 |
| Y | WO, 97/35851, A1 (Nippon Soda Co., Ltd.), 2 October, 1997 (02. 10. 97) & EP, 894792, A1 | 4-7 |

Form PCT/ISA/210 (continuation of second sheet) (July 1992)

## INTERNATIONAL SEARCH REPORT

International application No.
PCT/JP98/05571

**Box I Observations where certain claims were found unsearchable (Continuation of item 1 of first sheet)**

This international search report has not been established in respect of certain claims under Article 17(2)(a) for the following reasons:

1. ☐ Claims Nos.:
because they relate to subject matter not required to be searched by this Authority, namely:

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**Box II Observations where unity of invention is lacking (Continuation of item 2 of first sheet)**

This International Searching Authority found multiple inventions in this international application, as follows:

This invention is classified into (1) a group of inventions of claims 1 and 2 relating to processes for producing MgX-bearing toluene derivatives from Cl-bearing toluene derivatives, (2) a group of invention of claim 3 relating to a process for producing COOH-bearing toluene derivatives from MgX-bearing toluene derivatives, and (3) a group of inventions of claims 4-7 relating to processes for producing OR-bearing toluene derivatives from Cl-bearing toluene derivatives, and thus these three groups of inventions are not considered having a special technical feature in common.

Such being the case, the number of inventions of the international application as set forth in the claims is three.

1. ☒ As all required additional search fees were timely paid by the applicant, this international search report covers all searchable claims.

2. ☐ As all searchable claims could be searched without effort justifying an additional fee, this Authority did not invite payment of any additional fee.

3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest** ☐ The additional search fees were accompanied by the applicant's protest.

☒ No protest accompanied the payment of additional search fees.

Form PCT/ISA/210 (continuation of first sheet (1)) (July 1992)